# EUROPEAN PATENT APPLICATION

(11) **EP 4 778 556 A2**
(43) Date of publication of application: **22.07.2026**
(21) Application number: 26174811.5
(22) Date of filing: 06.03.2020
(51) Int. Cl.: A61M 15/06

(54) **SMOKING SUBSTITUTE SYSTEM**

(30) Priority: 29.03.2019 EP 19166304
(62) Divisional of application: 20708105.0
(71) Applicant: Imperial Tobacco Limited, Bristol BS3 2LL (GB)
(72) Inventor: LOMAS, Peter, Bristol, BS3 2LL (GB); LORD, Chris, Bristol, BS3 2LL (GB)
(74) Representative: Mewburn Ellis LLP

(57) **Abstract**

Disclosed a smoking substitute device comprising a body accommodating a power source, and one or more side walls projecting longitudinally from an end surface of the body. The end surface and the one or more side walls define a cavity for receipt of a vaporisable liquid reservoir and a vaporiser to vaporise the vaporisable liquid. The device further comprises an air inlet aperture formed in the one or more side walls, the aperture arranged so as to direct air that is external to the device into the cavity in a substantially transverse direction across the end surface. Also disclosed is a system comprising the smoking substitute device, a tank for storing vaporisable liquid, a vaporiser for vaporising the vaporisable liquid, the vaporiser mounted in a vaporising chamber, an airflow path extending substantially transversely from the air inlet aperture to an inlet of the vaporising chamber.

## Description

### Field of the Invention

The present invention relates to a smoking substitute device and a system including the device, and, more particularly but not exclusively, to a smoking substitute device for delivering an aerosol to a user.

### Background

The smoking of tobacco is generally considered to expose a smoker to potentially harmful substances. It is generally thought that a significant amount of the potentially harmful substances are generated through the heat caused by the burning and/or combustion of the tobacco and the constituents of the burnt tobacco in the tobacco smoke itself.

Combustion of organic material such as tobacco is known to produce tar and other potentially harmful by-products. There have been proposed various smoking substitute devices in order to avoid the smoking of tobacco.

Such smoking substitute systems can form part of nicotine replacement therapies aimed at people who wish to stop smoking and overcome a dependence on nicotine.

Smoking substitute systems, which may also be known as electronic nicotine delivery systems, may comprise electronic systems that permit a user to simulate the act of smoking by producing an aerosol, also referred to as a "vapour", which is drawn into the lungs through the mouth (inhaled) and then exhaled. The inhaled aerosol typically bears nicotine and/or flavourings without, or with fewer of, the odour and health risks associated with traditional smoking.

In general, smoking substitute systems are intended to provide a substitute for the rituals of smoking, whilst providing the user with a similar experience and satisfaction to those experienced with traditional smoking and tobacco products.

The popularity and use of smoking substitute systems has grown rapidly in the past few years. Some smoking substitute systems are designed to resemble a traditional cigarette and are cylindrical in form with a mouthpiece at one end. Other smoking substitute systems do not generally resemble a cigarette (for example, the smoking substitute device may have a generally box-like form).

There are a number of different categories of smoking substitute systems, each utilising a different smoking substitute approach. A smoking substitute approach corresponds to the manner in which the substitute system operates for a user.

One approach for a smoking substitute system is the so-called "vaping" approach, in which a vaporisable liquid, typically referred to (and referred to herein) as "e-liquid", is heated by a heater to produce an aerosol vapour which is inhaled by a user. An e-liquid typically includes a base liquid as well as nicotine and/or flavourings. The resulting vapour therefore typically contains nicotine and/or flavourings. The base liquid may include propylene glycol and/or vegetable glycerine.

A typical vaping smoking substitute system includes a mouthpiece, a power source (typically a battery), a tank or liquid reservoir for containing e-liquid, as well as a heater. In use, electrical energy is supplied from the power source to the heater, which heats the e-liquid to produce an aerosol (or "vapour") which is inhaled by a user through the mouthpiece.

Vaping smoking substitute systems can be configured in a variety of ways. For example, there are "closed system" vaping smoking substitute systems which typically have a heater and a sealed tank which is pre-filled with e-liquid and is not intended to be refilled by an end user. One subset of closed system vaping smoking substitute systems include a device which includes the power source, wherein the device is configured to be physically and electrically coupled to a consumable including the tank and the heater. In this way, when the tank of a consumable has been emptied, the device can be reused by connecting it to a new consumable. Another subset of closed system vaping smoking substitute systems are completely disposable, and intended for one-use only.

There are also "open system" vaping smoking substitute systems which typically have a tank that is configured to be refilled by a user, so the device can be used multiple times.

An example vaping smoking substitute system is the myblu^{™} e-cigarette. The myblu^{™} e cigarette is a closed system system which includes a device and a consumable. The device and consumable are physically and electrically coupled together by pushing the consumable into the device. The device includes a rechargeable battery. The consumable includes a mouthpiece, a sealed tank which contains e-liquid, as well as a vaporiser, which for this device is a heating filament coiled around a portion of a wick which is partially immersed in the e-liquid. The system is activated when a microprocessor on board the device detects a user inhaling through the mouthpiece. When the system is activated, electrical energy is supplied from the power source to the vaporiser, which heats e-liquid from the tank to produce a vapour which is inhaled by a user through the mouthpiece.

Another example vaping smoking substitute system is the blu PRO^{™} e-cigarette. The blu PRO^{™} e cigarette is an open system which includes a device, a (refillable) tank, and a mouthpiece. The device and tank are physically and electrically coupled together by screwing one to the other. The mouthpiece and refillable tank are physically coupled together by screwing one into the other, and detaching the mouthpiece from the refillable tank allows the tank to be refilled with e-liquid. The system is activated by a button on the device. When the system is activated, electrical energy is supplied from the power source to a vaporiser, which heats e-liquid from the tank to produce a vapour which is inhaled by a user through the mouthpiece.

In prior art smoking substitute systems, airflow through the system can be turbulent and can experience significant pressure drops. This can result in a user needing to provide a significant inhalation force to receive a suitable quantity of aerosol from the system.

The present invention has been devised in light of the above considerations. Additionally, it is desirable to provide consumables which are easier and cheaper to manufacture

### Summary of the Invention

At its most general, the present invention relates to a smoking substitute device having an air inlet arranged to provide a substantially linear/direct airflow path to a vaporiser for use with the device.

In a first aspect there is provided a smoking substitute device comprising:
a body accommodating a power source;
one or more side walls projecting longitudinally from an end surface of the body, such that the end surface and the one or more side walls define a cavity for receipt of a vaporisable liquid reservoir and a vaporiser to vaporise the vaporisable liquid; and
an air inlet aperture formed in the one or more side walls, the aperture arranged so as to direct air that is external to the device into the cavity in a substantially transverse direction across the end surface.

The provision of an air inlet that directs air transversely across the end surface provides a direct airflow path to the vaporiser. By providing a direct airflow path, as opposed to an airflow path that involves multiple turns/deflections, the pressure drop along the airflow path may be reduced. Similarly, turbulence in the air flow, which can be caused by turns/deflections in the airflow path, may be reduced by providing a more direct airflow. This may result in a reduction in the suction required by a user to draw air into the device and through the vaporiser.

The terms "transversely" and "transverse" are used herein to describe a direction that is substantially perpendicular to the axial (longitudinal) direction of the device.

Optional features of the present disclosure will now be set out. These are applicable singly or in any combination with any aspect of the present disclosure.

The end surface of the body (i.e. defining a base of the cavity) extends substantially transversely between the side wall(s). The aperture may be positioned so as to be proximate the end surface. For example, the aperture may be spaced from the end surface by a longitudinal distance that is less than 6 mm. The aperture the aperture may be spaced from the end surface by a longitudinal distance that is less than 4 mm, or less than 2 mm. An edge of the aperture may be longitudinally aligned with the end surface (i.e. there may be no longitudinal spacing between the aperture and the end surface).

The aperture may be circular, and may have a diameter of less than e.g. 2 mm. The aperture may alternatively be in the form of a slot. The slot may extend longitudinally or may be perpendicular to the longitudinal axis. The aperture (or slot) may have a cross-sectional area that is less than 10 mm² or less than 8 mm², or e.g. less than 5 mm².

The one or more side walls may comprise opposing front and rear walls (or wall portions) and opposing lateral walls (or wall portions) extending between the front and rear walls. The distance between the lateral walls may be greater than the distance between the front and rear walls. The distance between the lateral walls may be e.g. greater than twice the distance between the front and rear walls.

The aperture may be formed in any one of the front, rear, or lateral walls. The device may comprise two opposing apertures (i.e. each being as described above). For example, each of the lateral walls may comprise an aperture, or each of the front and rear walls may comprise an aperture.

The device may form part of an open smoking substitute system or a closed smoking substitute system. Where the device forms part of a closed smoking substitute system, the device may be configured for engagement (i.e. physical and electrical engagement) with a smoking substitute consumable. In such embodiments, a consumable may be received in the cavity. In that respect the side wall(s) may comprise an engagement portion for engaging a consumable. The engagement portion may be in the form of e.g. an inwardly extending detent or protrusion for engaging with a recess, lip, aperture, edge, etc. of a consumable.

The device may comprise an electrical interface for electrically interfacing with a consumable received in the cavity. The electrical interface may be electrically connected to the power source, so as to provide power from the power source to the consumable when interfaced therewith. The electrical interface may comprise two (e.g. transversely spaced) electrical contacts projecting longitudinally into the cavity from the end surface of the body (i.e. from the base of the cavity). The electrical contact(s) may project from or through a central portion of the end surface.

In a second aspect there is disclosed a smoking substitute system comprising:
a device according to the first aspect;
a tank for storing vaporisable liquid;
a vaporiser for vaporising the vaporisable liquid, the vaporiser mounted in a vaporising chamber; and
an airflow path extending substantially transversely from the air inlet aperture to an inlet of the vaporising chamber.

It should be appreciated that the term "substantially transverse" does not require the airflow path to extend exactly transversely. For example, the airflow path may be inclined by a small angle from a transverse axis (e.g. less than 25 degrees, or e.g. less than 15 degrees, or e.g. less than 5 degrees). Similarly a small portion of the airflow path may not be transverse (e.g. less than 10% of the length of the airflow path, or e.g. less than 5% of the length of the airflow path). In some embodiments, the airflow path may extend transversely (e.g. precisely transversely).

The air flow path may extend between the vaporiser (and/or the vaporiser chamber) and the end surface of the device. The air flow path may extend between the tank and the end surface of the device.

The air inlet aperture may be substantially longitudinally aligned (i.e. share a common transverse reference plane) with the vaporising chamber, vaporiser and/or the vaporising chamber inlet, when the consumable is received in the cavity. The aperture may be substantially longitudinally aligned with an upstream end of the consumable when the consumable is received in the cavity.

The vaporising chamber inlet may be oriented so as to be parallel to a transverse reference plane (i.e. so as to be perpendicular to the air inlet aperture). The vaporising chamber inlet may, for example, be formed in an upstream (e.g. lower transverse) wall of the vaporising chamber. In such embodiments, airflow from the transverse airflow path may be redirected at the inlet to a longitudinal direction. Only a single redirection (i.e. turn/deflection) of the airflow may be required between the air inlet aperture and the vaporiser). The term "upstream" is used to define a direction away from the outlet of the device. The term "downstream" is used to define a direction towards the outlet of the device.

In other embodiments, the vaporising chamber inlet may be oriented so as to be parallel to a longitudinal reference plane (i.e. so as to be parallel to the air inlet aperture). In such embodiments, the vaporising chamber inlet may, for example, be formed in a lateral wall (extending longitudinally between upstream and downstream walls) of the vaporising chamber.

The vaporising chamber may comprise a single (e.g. centrally located inlet), or a plurality of (e.g. two) inlets. Where the vaporising chamber comprises a plurality of inlets, the airflow path may extend to all of the vaporising chamber inlets (e.g. the airflow path may comprise branches or may comprise a plenum space).

The system may comprise a passage that extends from the vaporising chamber to an outlet of the device. An opening to the passage may be formed in a downstream transverse wall of the chamber.

A baffle may be interposed between the vaporiser and the passage. The baffle may extend generally transversely in the vaporising chamber and may be arranged such that un-vaporised liquid collects on an upstream (e.g. planar) surface of the baffle. The baffle may be aligned with the passage opening (from the vaporising chamber).

The outlet may form part of a mouthpiece of the device. The mouthpiece may be integrally formed with the passage (i.e. the one or more walls of the passage). The mouthpiece may define an outer surface of the device that is received in a user's mouth in use.

The vaporiser is in fluid communication with the tank so as to be in fluid communication with vaporisable liquid (e.g. e-liquid) in the tank. The e-liquid may, for example, may comprise a base liquid and e.g. nicotine. The base liquid may include propylene glycol and/or vegetable glycerine.

The tank may be defined by a tank housing. The tank housing may be integrally formed with the mouthpiece and/or the passage. At least a portion of the tank housing may be translucent. For example, the tank housing may comprise a window to allow a user to visually assess the quantity of e-liquid in the tank. The tank may be referred to as a "clearomizer" if it includes a window, or a "cartomizer" if it does not.

The passage may extend longitudinally within the tank and the one or more passage walls may define the inner wall of the tank. In this respect, the tank may surround the passage e.g. the tank may be annular. The passage wall(s) may comprise longitudinal ribs extending therealong. These ribs may provide support to the passage wall(s). The ribs may extend for the full length of the passage wall(s). The ribs may project (e.g. radially outwardly) into the tank.

The system may comprise an insert, which may define the vaporising chamber inlet. The insert may be inserted into an open end of the tank so as to seal against the tank housing. The insert may comprise an inner, longitudinally-extending sleeve that defines the wall(s) of the vaporising chamber and seals against the passage (e.g. seals against outer surfaces of the passage wall(s)). The insert may be configured to support the vaporiser within the vaporising chamber. The insert may be formed of silicone. The baffle may be formed of silicone. The insert and the baffle may be integrally formed.

The vaporiser may comprise a heater and a wick (e.g. comprising a porous material). The wick may be elongate and extend transversely across the chamber between wall(s) (e.g. side walls) of the chamber (which may be defined by the inner sleeve). So as to be in fluid communication with the tank, the wick extends into the tank, e.g. one or both of its opposing transverse ends may extend into the tank, e.g. through the wall(s) of the chamber/through the inner sleeve. In this way e-liquid may be drawn (e.g. by capillary action) along the wick, from the tank to the exposed (central) portion of the wick. The wick may be oriented so as to be perpendicular to the baffle. In this respect, air may pass around, through or proximate the wick and either side of the baffle.

The heater may comprise a heating element, which may be in the form of a filament wound about the wick (e.g. the filament may extend helically about the wick). The filament may be wound about the exposed portion of the wick. The heating element may be electrically connected (or connectable) to the power source. Thus, in operation, the power source may supply electricity to (i.e. apply a voltage across) the heating element so as to heat the heating element. This may cause liquid stored in the wick (i.e. drawn from the tank) to be heated so as to form a vapour and become entrained in the chamber portion of the airflow path. This vapour may subsequently cool to form an aerosol in the passage or vaporising chamber.

As is discussed above with respect to the first aspect, the system may be an open smoking substitute system or a closed smoking substitute system. Where the system is an open smoking substitute system the tank may be refillable, and the tank and vaporiser may be permanently mounted in the cavity defined by the side walls.

On the other hand, when the system is a closed smoking substitute system, the system may comprise a consumable. The consumable may include the tank, vaporising chamber (and vaporiser) and e.g. the passage. The consumable may be configured for engagement with the device (i.e. so as to form the smoking substitute system). For example, the consumable may comprise disposable components of the system, whilst the device may comprise non-disposable or non-consumable components (e.g. the power supply, a controller, a sensor, etc.) that facilitate the delivery of aerosol by the consumable. In such an embodiment, the vaporisable liquid may be replenished by replacing a used consumable with an unused consumable.

As is discussed above with respect to the first aspect, the device and the consumable may be configured to be physically coupled together. For example, the consumable may be at least partly received in the cavity defined by the side wall(s) of the device, such that there is snap engagement between the device and the consumable. Alternatively, the device and the consumable may be physically coupled together by screwing one onto the other, or through a bayonet fitting.

Thus, the consumable may comprise one or more engagement portions for engaging with the device. In this way, one end of the consumable (i.e. an upstream end comprising the vaporising chamber inlet) may be coupled with the device, whilst an opposing end (i.e. an outlet end) of the consumable may define the mouthpiece.

The airflow path (extending substantially transversely from the air inlet aperture) may extend between the consumable and the device when the consumable is received in the cavity. That is, the airflow path may extend between (and be defined by) the upstream (inlet) end (e.g. defined by the insert) of the consumable and the end surface of the body.

The end surface and/or the upstream end (e.g. underside) of the consumable may comprise guide portions that at least partly define the airflow path so as to guide airflow from the air inlet to the vaporising chamber inlet. The end surface and/or upstream end of the consumable may comprise a channel for directing air across the end surface. The device or the consumable may comprise spacer portions for spacing the upstream end (and/or inlet) of the consumable from the end surface of the body when the consumable is received in the cavity. The spacer portion may e.g. be a lip or seat (e.g. extending inwardly from the one or more side walls) on which the consumable is supported above the end surface.

The power source may be a battery (e.g. a rechargeable battery). An external electrical connector in the form of e.g. a USB port may be provided for recharging this battery. The consumable may comprise an electrical interface for interfacing with the electrical interface of the device. The electrical interface of the consumable may include one or more electrical contacts. Thus, when the device is engaged with the consumable, the electrical interface may be configured to transfer electrical power from the power source to the heater of the consumable. The electrical interface (of the device and/or consumable) may also be used to identify the consumable from a list of known types. The electrical interface (of the device and/or consumable) may additionally or alternatively be used to identify when the consumable is connected to the device.

The device may alternatively or additionally be able to detect information about the consumable via an RFID reader, a barcode or QR code reader. This (information) interface may be able to identify a characteristic (e.g. a type) of the consumable. In this respect, the consumable may include any one or more of an RFID chip, a barcode or QR code, or memory within which is an identifier and which can be interrogated via the information interface.

The device may comprise a controller, which may include a microprocessor. The controller may be configured to control the supply of power from the power source to the heater (e.g. via the electrical contacts). A memory may be provided and may be operatively connected to the controller. The memory may include non-volatile memory. The memory may include instructions which, when implemented, cause the controller to perform certain tasks or steps of a method.

The device may comprise a wireless interface, which may be configured to communicate wirelessly with another device, for example a mobile device, e.g. via Bluetooth^{®}. To this end, the wireless interface could include a Bluetooth^{®} antenna. Other wireless communication interfaces, e.g. WiFi^{®}, are also possible. The wireless interface may also be configured to communicate wirelessly with a remote server.

As is provided above, an airflow (i.e. puff) sensor may be provided that is configured to detect a puff (i.e. inhalation from a user). The airflow sensor may be operatively connected to the controller so as to be able to provide a signal to the controller that is indicative of a puff state (i.e. puffing or not puffing). The airflow sensor may, for example, be in the form of a pressure sensor or an acoustic sensor. The controller may control power supply to the heater in response to airflow detection by the sensor. The control may be in the form of activation of the heater in response to a detected airflow. The airflow sensor may form part of the consumable or the device.

The device may be an e-cigarette device and the consumable may be an e-cigarette consumable.

The invention includes the combination of the aspects and preferred features described except where such a combination is clearly impermissible or expressly avoided.

### Summary of the Figures

So that the invention may be understood, and so that further aspects and features thereof may be appreciated, embodiments illustrating the principles of the invention will now be discussed in further detail with reference to the accompanying figures, in which:
Figure 1A is a front schematic view of a smoking substitute system;
Figure 1B is a front schematic view of a device of the system;
Figure 1C is a front schematic view of a consumable of the system;
Figure 2A is a schematic of the components of the device;
Figure 2B is a schematic of the components of the consumable;
Figure 3A is a schematic section view of the device;
Figure 3B is a section view of the consumable engaged with the device; and
Figure 4 is a section view of a manufacturing assembly for manufacturing the consumable.

### Detailed Description of the Invention

Aspects and embodiments of the present invention will now be discussed with reference to the accompanying figures. Further aspects and embodiments will be apparent to those skilled in the art. All documents mentioned in this text are incorporated herein by reference.

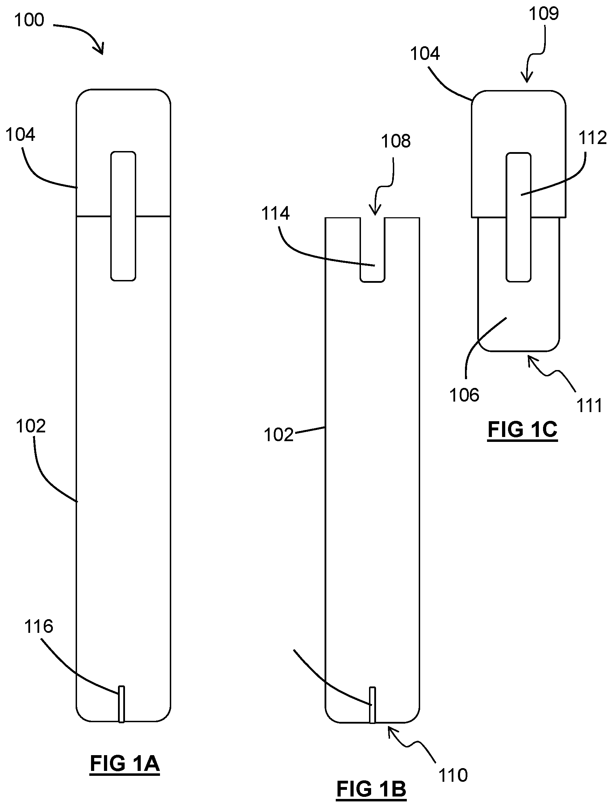

Figure 1A shows a first embodiment of a smoking substitute system 100. In this example, the smoking substitute system 100 includes a device 102 and a consumable 104. The consumable 104 may alternatively be referred to as a "pod", "cartridge" or "cartomizer". It should be appreciated that in other examples (i.e. open systems), the device may be integral with the consumable. In such (open) systems, a tank of the aerosol delivery device may be accessible for refilling the device.

In this example, the smoking substitute system 100 is a closed system vaping system, wherein the consumable 104 includes a sealed tank 106 and is intended for single-use only. The consumable 104 is removably engagable with the device 102 (i.e. for removal and replacement). Figure 1A shows the smoking substitute system 100 with the device 102 physically coupled to the consumable 104, Figure 1B shows the device 102 of the smoking substitute system 100 without the consumable 104, and Figure 1C shows the consumable 104 of the smoking substitute system 100 without the device 102.

The device 102 and the consumable 104 are configured to be physically coupled together by pushing the consumable 104 into a cavity (not shown in Figures 1A-1C) at an upper end 108 of the device 102, such that there is an interference fit between the device 102 and the consumable 104. In other examples, the device 102 and the consumable may be coupled by screwing one onto the other, or through a bayonet fitting.

The consumable 104 includes a mouthpiece (not shown in Figure 1A-1C) at an upper end 109 of the consumable 104, and one or more air inlets (again, not shown) in fluid communication with the mouthpiece such that air can be drawn into and through the consumable 104 when a user inhales through the mouthpiece. The tank 106 containing e-liquid is located at the lower end 111 of the consumable 104.

The tank 106 includes a window 112, which allows the amount of e-liquid in the tank 106 to be visually assessed. The device 102 includes a slot 114 so that the window 112 of the consumable 104 can be seen whilst the rest of the tank 106 is obscured from view when the consumable 104 is inserted into the cavity at the upper end 108 of the device 102.

The lower end 110 of the device 102 also includes a light 116 (e.g. an LED) located behind a small translucent cover. The light 116 may be configured to illuminate when the smoking substitute system 100 is activated. Whilst not shown, the consumable 104 may identify itself to the device 102, via an electrical interface, RFID chip, or barcode.

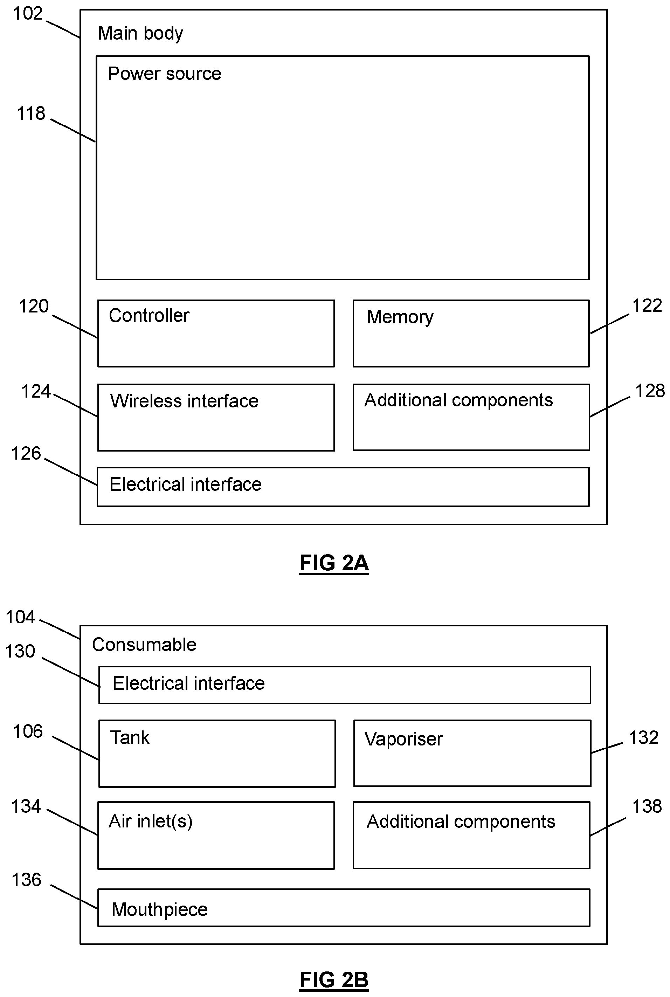

Figures 2A and 2B are schematic drawings of the device 102 and consumable 104. As is apparent from Figure 2A, the device 102 includes a power source 118, a controller 120, a memory 122, a wireless interface 124, an electrical interface 126, and, optionally, one or more additional components 128.

The power source 118 is preferably a battery, more preferably a rechargeable battery. The controller 120 may include a microprocessor, for example. The memory 122 preferably includes non-volatile memory. The memory may include instructions which, when implemented, cause the controller 120 to perform certain tasks or steps of a method.

The wireless interface 124 is preferably configured to communicate wirelessly with another device, for example a mobile device, e.g. via Bluetooth^{®}. To this end, the wireless interface 124 could include a Bluetooth^{®} antenna. Other wireless communication interfaces, e.g. WiFi^{®}, are also possible. The wireless interface 124 may also be configured to communicate wirelessly with a remote server.

The electrical interface 126 of the device 102 may include one or more electrical contacts. The electrical interface 126 may be located at a base of the cavity in the upper end 108 of the device 102. When the device 102 is physically coupled to the consumable 104, the electrical interface 126 is configured to transfer electrical power from the power source 118 to the consumable 104 (i.e. upon activation of the smoking substitute system 100).

The electrical interface 126 may be configured to receive power from a charging station when the device 102 is not physically coupled to the consumable 104 and is instead coupled to the charging station. The electrical interface 126 may also be used to identify the consumable 104 from a list of known consumables. For example, the consumable 104 may be a particular flavour and/or have a certain concentration of nicotine (which may be identified by the electrical interface 126). This can be indicated to the controller 120 of the device 102 when the consumable 104 is connected to the device 102. Additionally, or alternatively, there may be a separate communication interface provided in the device 102 and a corresponding communication interface in the consumable 104 such that, when connected, the consumable 104 can identify itself to the device 102.

The additional components 128 of the device 102 may comprise the light 116 discussed above.

The additional components 128 of the device 102 may also comprise a charging port (e.g. USB or micro-USB port) configured to receive power from the charging station (i.e. when the power source 118 is a rechargeable battery). This may be located at the lower end 110 of the device 102. Alternatively, the electrical interface 126 discussed above may be configured to act as a charging port configured to receive power from the charging station such that a separate charging port is not required.

The additional components 128 of the device 102 may, if the power source 118 is a rechargeable battery, include a battery charging control circuit, for controlling the charging of the rechargeable battery. However, a battery charging control circuit could equally be located in the charging station (if present).

The additional components 128 of the device 102 may include a sensor, such as an airflow (i.e. puff) sensor for detecting airflow in the system 100, e.g. caused by a user inhaling through a mouthpiece 136 of the consumable 104. The smoking substitute system 100 may be configured to be activated when airflow is detected by the airflow sensor. This sensor could alternatively be included in the consumable 104. The airflow sensor can be used to determine, for example, how heavily a user draws on the mouthpiece or how many times a user draws on the mouthpiece in a particular time period.

The additional components 128 of the device 102 may include a user input, e.g. a button. The smoking substitute system 100 may be configured to be activated when a user interacts with the user input (e.g. presses the button). This provides an alternative to the airflow sensor as a mechanism for activating the smoking substitute system 100.

As shown in Figure 2B, the consumable 104 includes the tank 106, an electrical interface 130, a vaporiser 132, one or more air inlets 134, a mouthpiece 136, and one or more additional components 138.

The electrical interface 130 of the consumable 104 may include one or more electrical contacts. The electrical interface 126 of the device 102 and an electrical interface 130 of the consumable 104 are configured to contact each other and thereby electrically couple the device 102 to the consumable 104 when the lower end 111 of the consumable 104 is inserted into the cavity at the upper end of the device 102 (as shown in Fig. 1A). In this way, electrical energy (e.g. in the form of an electrical current) is able to be supplied from the power source 118 of the device 102 to the vaporiser 132 of the consumable 104.

The vaporiser 132 is configured to heat and vaporise e-liquid contained in the tank 106 using electrical energy supplied from the power source 118. As will be described further below, the vaporiser 132 includes a heating filament and a wick. The wick draws e-liquid from the tank 106 and the heating filament heats the e-liquid to vaporise the e-liquid.

The one or more air inlet(s) 134 are preferably configured to allow air to be drawn into the consumable 104, when a user inhales through the mouthpiece 136. As will be discussed further below, when the consumable 104 is physically coupled to the device 102 air flows along an airflow path between the device 102 and the lower end 111 of the consumable 104 to the air inlet(s) of the consumable 104.

In operation, a user activates the smoking substitute system 100, e.g. through interaction with a user input forming part of the device 102 or by inhaling through the mouthpiece 136 as described above. Upon activation, the controller 120 may supply electrical energy from the power source 118 to the vaporiser 132 (via electrical interfaces 126, 130), which may cause the vaporiser 132 to heat e-liquid drawn from the tank 106 to produce a vapour which is inhaled by a user through the mouthpiece 136.

An example of one of the one or more additional components 138 of the consumable 104 is an interface for obtaining an identifier of the consumable 104. As discussed above, this interface may be, for example, an RFID reader, a barcode, a QR code reader, or an electronic interface which is able to identify the consumable. The consumable 104 may, therefore include any one or more of an RFID chip, a barcode or QR code, or memory within which is an identifier and which can be interrogated via the electronic interface in the device 102.

It should be appreciated that the smoking substitute system 100 shown in figures 1A to 2B is just one exemplary implementation of a smoking substitute system. For example, the system could otherwise be in the form of an entirely disposable (single-use) system or an open system in which the tank is refillable (rather than replaceable).

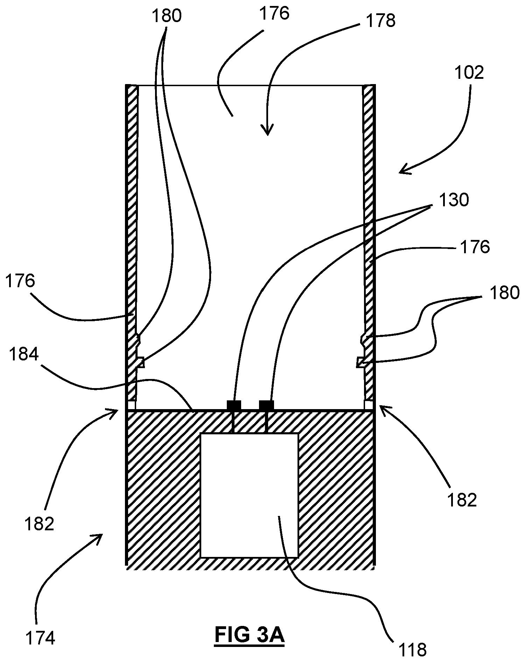

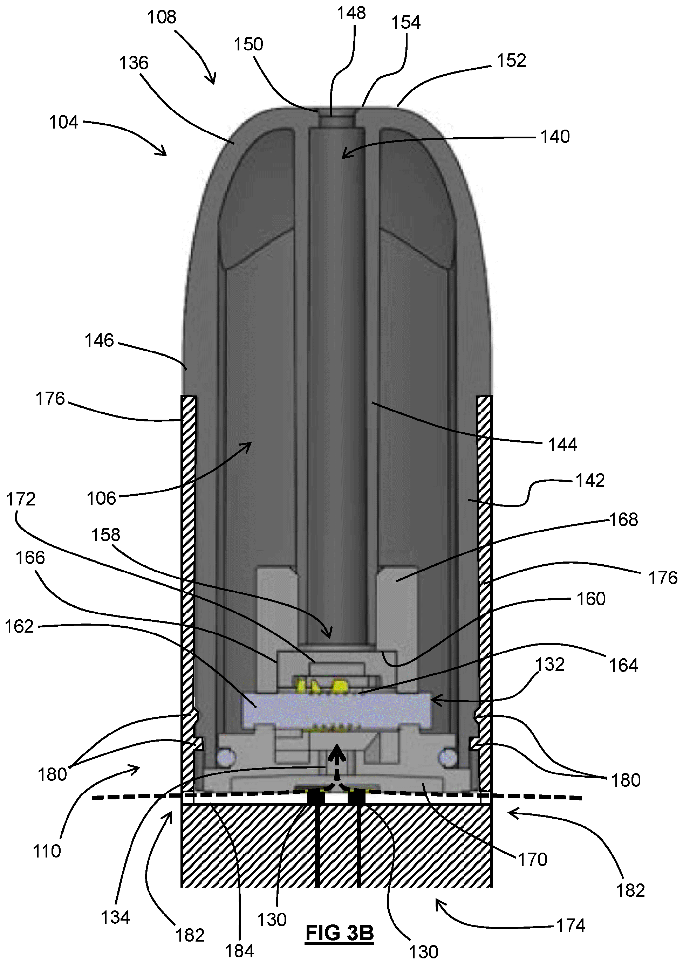

Figure 3A is a schematic section view of an upper end of the device 102. The device 102 comprises a body 174 accommodating the power source 118, and side walls 176. The side walls 176 extend (upwardly) from an end surface 184 of the body 174 in a longitudinal direction so as to define a cavity 178 for receipt of the consumable 104. In order to facilitate engagement with the consumable 104, two of the side walls 176 include two inwardly extending projections 180. These projections 180 engage with recesses of the consumable 104 when received in the cavity 178, so as to help retain the consumable 104 in the cavity 178. When engaged in this manner (as shown in Figure 3B), the consumable 104 interfaces with the electrical interface 130 (in the form of electrical contacts) of the device 102 such that power is supplied to the consumable 104 from the power supply 118 of the device 102.

Each of the side walls 176 comprises an air inlet aperture 182 formed therein. Each air inlet aperture 182 is formed in its respective side wall 176 at a (longitudinal) position that is adjacent the end surface 184 of the cavity 178. That is, a lower edge of each aperture 182 is aligned with the end surface 184 of the cavity 178. As will be discussed further below with response to Figure 3B, this positioning of the apertures 182 allows air (i.e. that is external to the device 102) to be directed into the cavity 178 in a substantially transverse direction and between body 174 (i.e. the end surface 184) and the consumable 104.

Figure 3B is a section view of the device 102 engaged with the consumable 104. The consumable 104 comprises a tank 106 for storing e-liquid, a mouthpiece 136 and a passage 140 extending along a longitudinal axis of the consumable 104. In the illustrated embodiment the passage 140 is in the form of a tube having a substantially circular transverse cross-section (i.e. transverse to the longitudinal axis). The tank 106 surrounds the passage 140, such that the passage 140 extends centrally through the tank 106.

A tank housing 142 of the tank 106 defines an outer casing of the consumable 104, whilst a passage wall 144 defines the passage 140. The tank housing 142 extends from the lower end of the consumable 111 to the mouthpiece 136 at the upper end 109 of the consumable 104. At the junction between the mouthpiece 136 and the tank housing 142, the mouthpiece 136 is wider than the tank housing 142, so as to define a lip 146 that overhangs the tank housing 142. This lip 146 acts as a stop feature when the consumable 104 is inserted into the device102 (i.e. by contact with an upper end of the side walls 176 of the device 102).

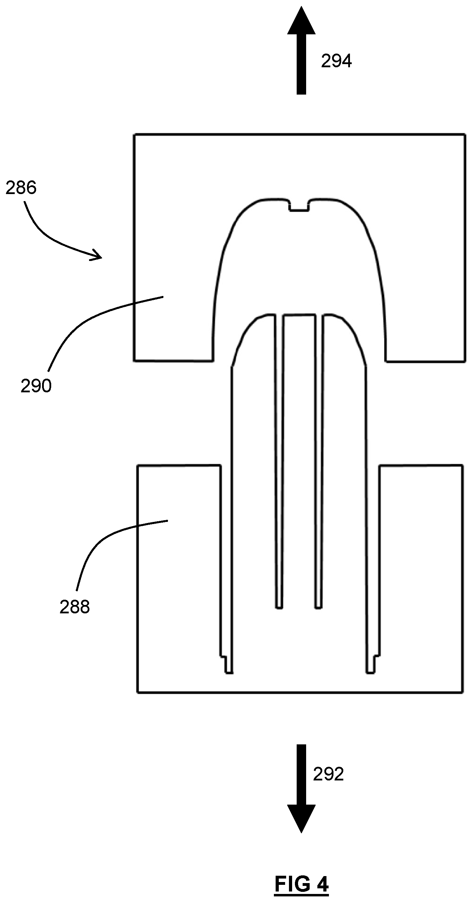

The tank 106, the passage 140 and the mouthpiece 136 are integrally formed with each other so as to form a single unitary component. As will be described further below with respect to Figure 4, this component may be formed by way of an injection moulding process and, for example, may be formed of a thermoplastic material such as polypropylene.

Although not immediately apparent from the figures, the tank housing 142 tapers, such that the thickness of the tank housing 142 decreases in a first demoulding direction (as will be discussed further with respect to Figure 4). In Figure 3A the first demoulding direction is in a downward direction away from the mouthpiece 136. This means that, aside from a small number of indents (which provide physical connection between the consumable 104 and the device 102), the thickness of the tank housing 142 decreases with increasing distance away from the mouthpiece 136. In particular, the tank housing 142 tapers in this way, because internal and external surfaces of the tank housing 142 are angled with respect to the first demoulding direction. This tapering assists in forming the tank housing 142 and passage wall 144 as a single (i.e. unitary) component.

Like the tank housing 142, the passage wall 144 is also tapered such that the thickness of the passage wall 144 decreases along the first demoulding direction. Again, the thickness of the passage wall 144 decreases due to internal and external surfaces of the passage wall 144 being angled with respect to the first demoulding direction. As a result of the tapering of the passage wall 144, the passage 140 has an internal diameter that decreases in a downstream direction (i.e. an upward direction in Figure 3B). For example, the passage 140 has an internal width less than 4.0 mm and greater than 3.0 mm at an upstream end of the passage 140 (e.g. approximately 3.6 mm). On the other hand, the passage 140 has an internal width of less than 3.8 mm and greater than 2.8 mm at the downstream end of the passage 140 (e.g. approximately 3.4 mm).

The mouthpiece 136 comprises a mouthpiece aperture 148 defining an outlet of the passage 140. The mouthpiece aperture 148 has a radially inwardly directed inner surface 150, which joins an outer surface 152 of the mouthpiece 136 (i.e. a surface which contacts a user's lips in use) at an outer edge 154 of the mouthpiece aperture 148. At this outer edge 154, the included angle between the inner surface 150 of the mouthpiece aperture 148 and the outer surface 152 of the mouthpiece 136 (i.e. the "mouthpiece angle") is greater than 90 degrees. In the illustrated embodiment, this is due to the outer edge 154 being rounded. This edge 154 may otherwise be chamfered or bevelled.

The vaporiser 132 is located in a vaporising chamber 156 of the consumable 104. The vaporising chamber 158 is fluidly connected to the mouthpiece aperture 148 (i.e. outlet) by the passage 140. In particular, the passage 140 extends between the mouthpiece aperture 148 and an opening 158 from the chamber 156. This opening 158 is formed in a downstream (i.e. upper) wall 160 of the chamber 156.

The inlet 134 of the consumable 104 (as discussed above) is an inlet 134 to the vaporising chamber. This inlet 134 is fluidly connected to each of the air inlet apertures 182 via respective airflow paths that extend transversely across the end surface 184 of the body 174. In particular, these airflow paths are defined between the body 174 (i.e. the end surface 184) and the consumable 104 (i.e. the insert 170). That is, when the consumable 104 is received in the cavity 178, a gap is maintained between the insert 170 and the end surface 184. This gap is aligned with the air inlet apertures 182 to permit transverse airflow therethrough. Whilst not shown, the end surface 184 or the consumable 104 may comprise guide surfaces (e.g. ribs) that project therefrom so as to at least partly define the airflow path (e.g. to define the transverse path that the airflow takes).

The vaporiser 132 comprises a porous wick 162 and a heater filament 164 coiled around the porous wick 162. The wick 162 extends transversely across the chamber 156 between sidewalls 166 of the chamber 156 which form part of an inner sleeve 168 of an insert 170 that defines the lower end 111 of the consumable 104 (i.e. that connects with the device 102). The insert 170 is inserted into an open lower end of the tank 106 so as to seal against the tank housing 142.

In this way, the inner sleeve 168 projects into the tank 106 and seals with the passage 140 (around the passage wall 144) so as to separate the chamber 156 from the e-liquid in the tank 106. Ends of the wick 162 project through apertures in the inner sleeve 168 and into the tank 106 so as to be in contact with the e-liquid in the tank 106. In this way, e-liquid is transported along the wick 162 (e.g. by capillary action) to a central portion of the wick 162 that is exposed to airflow through the chamber 156. The transported e-liquid is heated by the heater filament 164 (when activated e.g. by detection of inhalation), which causes the e-liquid to be vaporised and to be entrained in air flowing past the wick 162. This vaporised liquid may cool to form an aerosol in the passage 140, which may then be inhaled by a user.

In some cases, un-vaporised liquid can be carried by air flowing through the chamber 156. This may be undesirable for a user. To reduce this, the consumable 104 comprises a baffle 172. The baffle 172 extends across the chamber 156 so as to be interposed between the vaporiser 132 and the passage opening 158. In this way, un-vaporised liquid from the wick 162 may collect on an upstream (i.e. lower) planar surface of the baffle 172 rather than entering the passage 158.

Figure 4 shows a drawing of a manufacturing assembly 286 which is used to manufacture the consumable 104 described above. The manufacturing assembly 286 comprises a first mould 288 and a second mould 290.

The first mould 288 has a shape which complements that of a first end of the integrally formed tank housing 142 and mouthpiece 136. The first mould 288 therefore has a shape which matches the inner surfaces defining the tank 106.

The second mould 290 has a shape which complements that of a second end of the integrally formed tank housing 142 and mouthpiece 136. The second mould 290 has a shape which matches the outer surface of the mouthpiece 136 and the inner surface of the mouthpiece aperture 148.

When the first mould 288 and the second mould 290 are brought together, they define a closed cavity which has the shape of the tank housing 142, the mouthpiece 136 and the passage walls 144.

To manufacture these components, heated material is injected into the cavity between the first mould 288 and the second mould 290. At this point, the first mould 288 and the second mould 290 meet at a boundary between external surfaces of the mouthpiece 136 and the tank housing 142.

The material is subsequently cooled, and the first mould 288 and the second mould 290 are separated, with the first mould 288 travelling in a first demoulding direction 292 (i.e. away from the second mould 290) and the second mould 290 travelling in a second demoulding direction 294 (i.e. away from the first mould 288 and opposite to the first demoulding direction 292). For a particular component, a demoulding direction is a direction along which a mould which contacts that component is removed during an injection moulding process.

Subsequently, the insert 170 (e.g. including the vaporiser) and any additional components are inserted into the tank 106 to form the consumable 104.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the words "have", "comprise", and "include", and variations such as "having", "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means, for example, +/- 10%.

The words "preferred" and "preferably" are used herein refer to embodiments of the invention that may provide certain benefits under some circumstances. It is to be appreciated, however, that other embodiments may also be preferred under the same or different circumstances. The recitation of one or more preferred embodiments therefore does not mean or imply that other embodiments are not useful, and is not intended to exclude other embodiments from the scope of the disclosure, or from the scope of the claims.

Features of embodiments of the invention are set out in the following paragraphs.

Clause 1. A smoking substitute device comprising: a body accommodating a power source; one or more side walls projecting longitudinally from an end surface of the body, such that the end surface and the one or more side walls define a cavity for receipt of a vaporisable liquid reservoir and a vaporiser to vaporise the vaporisable liquid; and an air inlet aperture formed in the one or more side walls, the aperture arranged so as to direct air that is external to the device into the cavity in a substantially transverse direction across the end surface.

Clause 2. A smoking substitute device according to clause 1 wherein the aperture is positioned so as to be proximate to the end surface.

Clause 3. A smoking substitute device according to clause 2 wherein the aperture is spaced from the end surface by a longitudinal distance that is less than 4 mm.

Clause 4. A smoking substitute device according to clause 3 wherein the aperture is spaced from the end surface by a longitudinal distance that is less than 2 mm.

Clause 5. A smoking substitute device according to clause 4 wherein an edge of the aperture is longitudinally aligned with the end surface.

Clause 6. A smoking substitute system according to any one of the preceding clauses comprising a two air inlet apertures opposing one another so as to direct air into the cavity in opposing substantially transverse directions.

Clause 7. A smoking substitute device according to any one of clauses 1 to 5 wherein the one or more side walls comprises opposing front and rear walls and opposing lateral walls extending between the front and rear walls, the distance between the lateral walls being greater than the distance between the front and rear walls, and wherein the aperture is formed in one of the lateral walls.

Clause 8. A smoking substitute device according to clause 7 comprising a first air inlet aperture in a first of the lateral walls and a second inlet air aperture in a second of the lateral walls.

Clause 9. A smoking substitute device according to any one of the preceding clauses that is an e-cigarette device.

Clause 10. A smoking substitute system comprising: a smoking substitute device according to any one of the preceding clauses; a tank for storing vaporisable liquid; a vaporiser for vaporising the vaporisable liquid, the vaporiser mounted in a vaporising chamber; and an airflow path extending substantially transversely from the air inlet aperture to an inlet of the vaporising chamber.

Clause 11. A smoking substitute system according to clause 10 wherein the airflow path extends between the vaporiser and the end surface of the device.

Clause 12. A smoking substitute system according to clause 10 or 11 comprising a consumable engageable with the device by receipt in the cavity of the device, and wherein the tank, vaporiser and vaporising chamber form part of the consumable.

Clause 13. A smoking substitute system according to clause 12 wherein the airflow path is at least partly defined between an upstream end of the consumable and the end surface of the device.

Clause 14. A smoking substitute system according to clause 12 or 13 wherein the device or the consumable comprises a spacer portion configured to space the upstream end of the consumable from the end surface of the device when the consumable is engaged with the device.

Clause 15. A smoking substitute system according to any one of clauses 10 to 14 wherein the consumable is an e-cigarette consumable.

## Claims

1. A smoking substitute device comprising:
a body accommodating a power source;
one or more side walls projecting longitudinally from an end surface of the body, such that the end surface and the one or more side walls define a cavity for receipt of a vaporisable liquid reservoir and a vaporiser to vaporise the vaporisable liquid; and
an air inlet aperture formed in the one or more side walls, the aperture arranged so as to direct air that is external to the device into the cavity in a substantially transverse direction across the end surface.

2. The smoking substitute device according to claim 1 wherein the aperture is spaced from the end surface by a longitudinal distance that is less than 4 mm, optionally less than 2 mm, optionally wherein an edge of the aperture is longitudinally aligned with the end surface.

3. The smoking substitute device according to claim 1 or 2 wherein the one or more side walls comprises opposing front and rear walls and opposing lateral walls extending between the front and rear walls, the distance between the lateral walls being greater than the distance between the front and rear walls, and wherein the aperture is formed in one of the lateral walls.

4. The smoking substitute device according to claim 3, wherein the distance between the lateral walls is greater than twice the distance between the front and rear walls.

5. The smoking substitute device according to claim 3 or 4 comprising a first air inlet aperture in a first of the lateral walls and a second inlet air aperture in a second of the lateral walls.

6. The smoking substitute device according to any preceding claim, wherein the one or more side walls comprise an engagement portion configured to engage a consumable when the consumable is received in the cavity, the engagement portion comprising an inwardly extending detent or protrusion for engaging with a recess, lip, aperture, or edge of the consumable.

7. The smoking substitute device according to any preceding claim, wherein the device comprises an electrical interface for electrically interfacing with the consumable received in the cavity, the electrical interface being electrically connected to the power source and comprising two electrical contacts projecting longitudinally into the cavity from the end surface of the body.

8. A smoking substitute system comprising:
a smoking substitute device according to any one of the preceding claims;
a tank defining the vaporisable liquid reservoir for storing vaporisable liquid;
the vaporiser for vaporising the vaporisable liquid, the vaporiser mounted in a vaporising chamber; and
an airflow path extending substantially transversely from the air inlet aperture to an inlet of the vaporising chamber.

9. The smoking substitute system according to claim 8, wherein the airflow path defines only a single redirection of airflow between the air inlet aperture and the inlet of the vaporising chamber.

10. The smoking substitute system according to claim 8 or 9 comprising a consumable engageable with the device by receipt in the cavity of the device, and wherein the tank, vaporiser and vaporising chamber form part of the consumable.

11. The smoking substitute system of claim 10, wherein an end surface of the consumable comprises a channel that at least partly defines the airflow path so as to guide airflow from the air inlet to the vaporising chamber inlet.

12. The smoking substitute system of claim 10 or 11, wherein the consumable comprises a passage that extends from the vaporising chamber to an outlet that forms part of a mouthpiece, wherein the passage extends centrally through the tank.

13. The smoking substitute system of claim 12, wherein the mouthpiece is integrally formed with the passage so as to form a single unitary component.

14. The smoking substitute system of claim 12 or 13, comprising a baffle which is interposed between the vaporiser and the passage.

15. The smoking substitute system according to claim 8 or 9, wherein the liquid reservoir and vaporiser are permanently mounted in the cavity.
